# EUROPÄISCHE PATENTSCHRIFT

(11) **EP 1 909 885 B1**
(45) Veröffentlichungstag und Bekanntmachung des Hinweises auf die Patenterteilung: **16.09.2009**
(21) Anmeldenummer: 06776377.1
(22) Anmeldetag: 24.07.2006
(51) Int. Cl.: A61M 25/10, A61F 2/06

(54) **Ballondilationskatheter**
Balloon dilatation catheter
Cathéter de dilatation à ballonnet

(30) Priorität: 23.07.2005 DE 102005034529
(43) Veröffentlichungstag der Anmeldung: 16.04.2008
(73) Patentinhaber: Qualimed Innovative Medizinprodukte GmbH, 21423 Winsen (DE)
(72) Erfinder: NIBL, Thomas, 21441 Garstedt (DE)
(74) Vertreter: Thiel, Christian
(86) Internationale Anmeldenummer: PCT/EP2006/007266
(87) Internationale Veröffentlichungsnummer: WO 2007/012443

(56) Entgegenhaltungen:
- WO-A-89/11307
- WO-A-99/20324
- WO-A-99/27989
- WO-A-20/05082446
- WO-A2-02/22198
- US-A- 5 156 620
- US-A- 5 181 911
- US-A- 5 370 691
- US-A- 5 470 314
- US-A- 5 649 978
- US-A1- 2002 151 942

## Beschreibung

Die Erfindung betrifft einen Ballondilatationskatheter, insbesondere für die perkutane transluminale Coronar-Angioplastie (PTCA), mit einem Führungsdraht, einem parallel zum Führungsdraht verlaufenden Versorgungsschlauch zum Aufpumpen eines Dilatationsballons sowie dem im distalen Bereich des Versorgungsschlauchs angeordneten Dilatationsballon.

Der erfindungsgemäße Ballondilatationskatheter ist in erster Linie für die perkutane transluminale Coronar-Angioplastie (PCTA) bestimmt, kann aber auch zur Aufweitung anderer, insbesondere peripherer Blutgefäße verwandt werden. Des Weiteren besteht auch die Möglichkeit, ihn in anderen Körperlumina einzusetzen.

Ballondilatationskatheter werden vielfach zur Behandlung von Strikturen, Stenosen oder Verengungen in verschiedenen Teilen des menschlichen Körpers eingesetzt. Derartige Ballonkatheter finden beispielsweise Anwendung in der perkutanen transluminalen Angioplastie (PTA) und insbesondere im Coronarbereich (PTCA), wo sie zur Beseitigung von Gefäßverengungen verwandt werden. Dazu wird über einen intravaskulär verlaufenden Katheter ein Dilatationsballon an die verengte Stelle geführt und dort hydraulisch über einen Versorgungsschlauch aufgepumpt, um die Verengung aufzuweiten und vorhandene Ablagerungen bei der Aufweitung so zu verdichten, dass nach Entfernung des Ballons das Gefäß mit einem zumindest stark aufgeweiteten Lumen verbleibt, wenn es auch in der Regel nicht möglich ist, die ursprünglich vorhandene Gefäßweite völlig wiederherzustellen.

Ballontechniken zur Dilatation eines Gefäßes stehen insoweit in Konkurrenz zu intraluminalen Stents, die ebenfalls zur Gefäßaufweitung dienen und in der Regel implantiert bleiben und so die Aufweitung dauerhaft fixieren. Die häufig fehlende Dauerhaftigkeit der Aufweitung ist ein zentraler Nachteil bei der Behandlung von Stenosen mit Ballonkathetem.

Grund für die fehlende Nachhaltigkeit der Aufweitung mit einem Dilatationsballon ist die Tendenz von Körpergefäße, sich nach Wegfall des vom Ballon ausgeübten Drucks wieder zusammenzuziehen. Dies liegt vor allem an der geringen Zeitdauer der Einwirkung eines Dilatationsballons. Da Dilatationsballons herkömmlicher Bauart zu einem mehr oder weniger vollständigen Gefäßverschluss führen, ist aber eine größere Nachhaltigkeit, d. h. eine längere Einwirkzeit, in der Regel nicht zu verwirklichen. Dies ist einer der Gründe, warum behandelnde Ärzte die Platzierung von Stents der Ballondilatation häufig verziehen.

Andererseits verbleiben Stents nach der Behandlung als Fremdkörper im Gefäß, während Dilatationsballons wieder entfernt werden.

Es wurde versucht, Katheter mit einem größeren Lumen zu verwenden, die eine gewisse Flussmöglichkeit zulassen. Diese Katheter lassen in der Regel aber nur geringe Blutmengen durch. Katheter mit Blutpumpen sind ebenfalls entwickelt worden, haben sich aber als aufwendig und teuer erwiesen, wobei die geförderte Blutmenge immer noch gering bleibt und zusätzlich die Gefahr der Blutschädigung besteht.

Der Erfindung liegt die Aufgabe zugrunde, ein Ballonsystem zur Gefäßaufweitung bereitzustellen, dass es erlaubt, bei gleichzeitiger Aufweitung einer Gefäßwand einen hinreichenden Blutfluss durch die Aufweitungsstelle aufrechtzuerhalten, um eine längere Einwirkzeit des Systems zu ermöglichen. Entsprechendes gilt für den Einsatz in anderen Körperlumina.

Aus der US 5,649,978 A geht eine Vorrichtung für die Ballonangioplastie hervor, bei der ein helikal gewundener Ballon expandiert wird, so dass ein inneres Lumen frei bleibt. Bei Implantation in das Gefäßsystem bleibt das Zentrum für den Blutfluss frei. Die Schlauchhelix wird proximal über einen Versorgungsschlauch mit Fluid versorgt.

Die WO 89/11307 A1 beschreibt einen Ballon für die Angioplastie, der im expandierten Zustand den Blutfluss ermöglicht. Der Ballon besteht aus einem expandierbaren gestreckten Schlauchsegment, das zylindrisch ausgebildet ist und im Querschnitt eine Donut-Form hat.

WO 99/27989 A1 beschreibt eine Vorrichtung zur Abgabe von Arzneimitteln in das Gefäßsystem. Die Vorrichtung besteht aus einem Ballon mit innerem Hohlraum, wobei es sich auch um mehrere beabstandete, miteinander verbundenen Zellen handeln kann, die durch Zufuhr eines Fluids expandierbar und im Gefäß verankerbar sind. Zur Abgabe des Medikaments ist die Ballonmembran mit Mikroporen versehen. Eine Eignung als Dilatationsballon ist bei dieser Vorrichtung weder angesprochen noch gegeben.

Die Erfindung betrifft einen Ballondilatationskatheter mit den in Anspruch 1 angegebenen Merkmalen. Bevorzugte Ausführungsformen sind Gegenstand der Unteransprüche.

Der erfindungsgemäße Ballondilatationskatheter unterscheidet sich von herkömmlichen durch die besondere Ausgestaltung des Ballonteils. Im übrigen ist der erfindungsgemäße Katheter in an und für sich bekannter Weise ausgebildet.

Die schlauchförmige Ausbildung des Dilatationsballons wird dadurch erreicht, dass die Wandung von einer Vielzahl von aneinandergrenzenden Schlauchsegmenten gebildet wird. Diese Schlauchsegmente werden über den Versorgungsschlauch mit Druckmedium versorgt, wobei alle Schlauchsegmente mit dem Versorgungsschlauch in Verbindung stehen und individuell mit Druckmedium beaufschlagt werden. Kombinationen der beiden Varianten sind ebenfalls möglich.

Die Schlauchsegmente der Ballonwandung sind im Wesentlichen parallel ausgerichtet und bilden eine innere Oberfläche, die dem verbleibenden Kanal bzw. zentralen Durchlass definieren sowie eine äußere Oberfläche, die gegen die Gefäßwand drückt und diese, nach Beaufschlagung mit dem Druckmedium, aufweitet.

Die Schlauchsegmente stehen an den Kontaktlinien miteinander in Verbindung um eine weitgehend homogene äußere Oberfläche des Ballons auszubilden. Dazu sind sie an den Kontaktlinien miteinander verklebt oder verschweißt. Über die Verbindung ist eine gleichmäßige Aufweitung des Ballons bei der Beaufschlagung mit Druck gewährleistet.

Allgewmein ist der erfindungsgemäß zum Einsatz kommende Dilatationsballon so ausgebildet, dass die Schlauchsegmente ringförmig ausgebildet sind. Sie verlaufen parallel und gehen im Wesentlichen senkrecht von dem zentralen Versorgungsschlauch aus. Dabei ist jedes einzelne Schlauchsegment an den Versorgungsschlauch angeschlossen und verläuft ringförmig, vom Versorgungsschlauch ausgehend zu selbigem zurück. Ein beidseitiger Anschluss an den Versorgungsschlauch ist nicht erforderlich, jedoch ist es zweckmäßig, das jeweils tote Ende eines Schlauchsegments mit dem Versorgungsschlauch zu verbinden, insbesondere zu verschweißen.

Der erfindungsgemäße Katheter kann gemäß einer weiteren Ausführungsform in seinem Innenraum einen inneren Ballon aufweisen, dergestalt, dass der schlauchförmig ausgebildete Dilatationsballon um diesen inneren Ballon herumgeführt ist. Dieser innere Ballon ist an einem separaten Versorgungsschlauch angeschlossen und kann separat geführt werden.

Dieser zweite oder innere Ballon dient als Expansionshilfe für den schlauchförmig ausgebildeten Ballon, insbesondere um Einsenkungen oder Einknickungen während des Dilatationsprozesses zu vermeiden. Der innere Ballon wird dazu parallel zur Expansion des Schlauchballons expandiert, kann jedoch auch vorexpandiert werden oder nachträglich zur Korrektur einer unvollständigen Entfaltung des Schlauchballons verwandt werden. Nach vollständiger Expansion des Schlauchballons wird der innere Ballon zurückgezogen, beispielsweise über einen separaten Führungsdraht oder über den Versorgungsschlauch. Durch Zurückziehen des inneren Ballons wird der Innenraum des schlauchförmigen Dilatationsballons für den Blutfluss frei.

Der erfindungsgemäße Katheter verfügt über einen im Wesentlichen konventionellen Führungsdraht, der innerhalb des Katheters parallel zum Versorgungsschlauch und innerhalb des schlauchförmigen Dilatationsballons verläuft. Vorzugsweise ist er an der Innenwandung des Ballons festgelegt, besonders bevorzugt an der der Versorgungsleitung gegenüberliegenden Seite der Balloninnenwand.

Ballon und/oder Führungsdraht können auf übliche Art und Weise proximal und/oder distal mit Markern versehen sein. In der Regel ist es zweckmäßig, die Marker am Führungsdraht anzuordnen dergestalt, dass über die Marker der Ballon exakt positioniert werden kann.

Katheter, Führungsdraht, Versorgungsschlauch sowie die Ballone bestehen aus den dafür üblicherweise eingesetzten Materialien. Dies sind für den Schlauchballon und den inneren Ballon in der Regel PET, Polyamid, Polyolefin, Polyurethan oder Polyester, wobei PET und Polyamid bevorzugt sind. Es ist zweckmäßig, den Ballon deutlich elastischer und dehnbarer auszulegen als den Versorgungsschlauch, jedoch kann die Stabilisierung der Versorgungsschlauchwandung gegen die Druckeinwirkung auch durch einen besonders druckfesten Katheter erfolgen.

Der erfindungsgemäße Ballondilatationskatheter ist insbesondere für die perkutane transluminale Coronar-Angiopiastie geeignet, kann aber auch zur Aufweitung anderer Blutgefäße eingesetzt werden. Darüber hinaus kann er in der Urologie in den Bronchien, bei Darmerkrankungen, in der Speiseröhre, im Gallengang und in der Luftröhre eingesetzt werden. Handhabung und Anwendungsbedingungen entsprechen denen herkömmlicher Ballonkatheter.

Der Vorteil des erfindungsgemäßen Ballondilatationskatheters liegt darin, dass er für eine längere Zeit im Gefäß belassen werden kann, ohne dessen Funktion grundlegend zu blockieren. Auf diese Weise können Blutgefäße über einen längeren Zeitraum, mehrere Minuten bis mehrere Stunden aufgeweitet werden, wodurch die Tendenz des Körpergefäßes zur Kontraktion nach Entfernung des Ballons entgegengewirkt wird. Insoweit hat der erfindungsgemäß zum Einsatz kommende Schlauchballon die Funktion und den Effekt eines temporären Stents.

Der Schlauchballon des erfindungsgemäßen Ballondilatationskatheters kann, bei geeigneter Ausführung, auch als Stent eingesetzt werden. Dazu ist es erforderlich, dass der Ballondilatationskatheter im unmittelbar an den Schlauchballon angrenzenden Teil des Versorgungsschlauches mit einem Einwegventil ausgestattet ist, dass das irreversible Aufpumpen des Schlauchballons erlaubt. Hat der Ballon seine Zielgröße erreicht, wird die Flüssigkeitszufuhr beendet und der Versorgungsschlauch unmittelbar proximal zum Einwegventil gelöst. Dies kann beispielsweise durch Zertrennen mittels einer heißen Drahtschlinge, über eine Bajonett- oder Schraubverbindung oder eine aktivierbare Sollbruchstelle erfolgen.

Ein derartiger isolierter Schlauchballon kann beispielsweise zum Aufweiten von Körperlumina verwandt werden, die mit herkömmlichen Stents nicht aufgeweitet werden können. Der Ballon fungiert als Stent, der aufgrund seiner flexiblen, weichen Oberfläche nur gemäßigten Druck auf das umgebende Gewebe ausübt. Er ist deshalb geeignet, auch im Bereich von gutartigen Tumoren und Wucherungen eingesetzt zu werden, für die herkömmliche Stents kontraindiziert sind.

Zur Entfernung eines solchen als Stent genutzten Schlauchballons ist es ausreichend, ihn anzustechen und die druckfreie Hülle mittels eines Retrievers zu bergen.

Entsprechend betrifft die Erfindung auch einen Schlauchballon, wie er nach Ablösung von dem erfindungsgemäßen Ballondilatationskatheter, wie vorstehend beschrieben, als Stent in einem Körperlumen platziert werden kann. Ein solcher Schlauchballon weist alle Merkmale auf, wie sie hier im Zusammenhang mit dem erfindungsgemäßen Ballondilatationskatheter beschrieben sind. Natürlich weist ein solcher Schlauchballon zusätzlich das vorstehend beschriebene Einwegventil auf und ggf. den Ansatz des abgetrennten Versorgungsschlauches. Insoweit kann zur Beschreibung des Schlauchballons auf die hier gegebene Beschreibung des Katheter gebundenen Schlauchballons verwiesen werden.

Die Erfindung wird durch die beigefügten Abbildungen näher erläutert. Von diesen zeigt:
- Fig. 1:: Eine erste Ausführungsform eines Schlauchballons gemäß der Erfindung;
- Fig. 2:: eine schematische Schnittzeichnung durch einen Schlauchballon gemäß Fig. 1 in Längsrichtung;
- Fig. 3:: eine weitere Schnittzeichnung senkrecht zum Verlauf des Versorgungsschlauchs;
- Fig. 4:: eine zweite Variante eines Schlauchballons gemäß der Erfindung mit einem inneren Ballon;
- Fig. 5:: einen Querschnitt durch den erfindungsgemäßen Schlauchballon gemäß Fig. 5;
- Fig. 6:: eine dritte Variante eines Schlauchballons gemäß der Erfindung mit einem Innenballon; und
- Fig. 7:: einen Querschnitt durch Ballon gemäß Fig. 7.

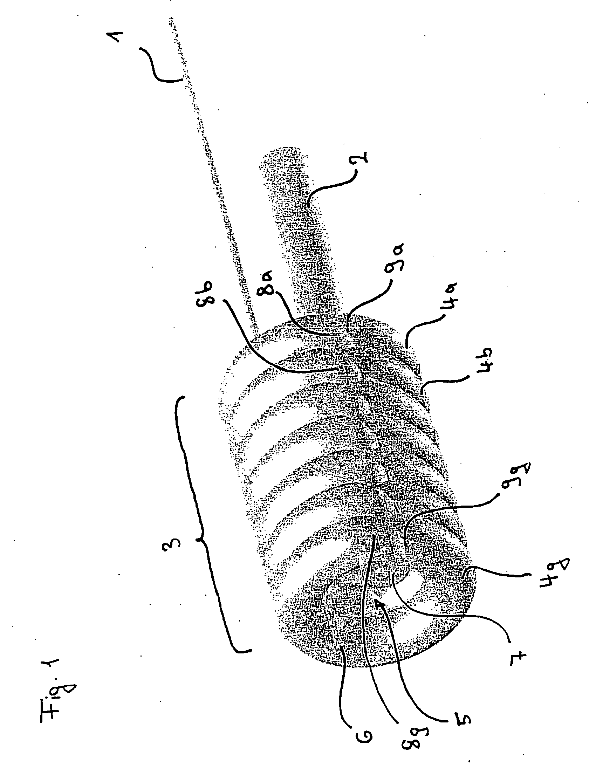

Figur 1 zeigt eine erste Ausführungsform der Erfindung, wobei in der Darstellung der (herkömmliche) Katheter nicht gezeigt wird.

Gezeigt sind der Führungsdraht 1 mit seinem distalen Ende 6 sowie der Versorgungsschlauch 2, der zur Druckbeaufschlagung des Ballons (3) mittels einer hydraulischen Pumpe durch den nicht gezeigten Katheter dient. Der Versorgungsschlauch 2 ist an seinem distalen Ende 7 verschlossen.

Der Dilatationsballon 3 ist schlauchförmig ausgebildet und bietet einen inneren Durchgang oder Hohlraum 5, der insbesondere bei der Aufweitung des Ballons frei bleibt und für Körperflüssigkeiten passierbar ist. Die Wandung des Schlauchballons 3 wird aus einer Mehrzahl von Schlauchsegmenten 4 (4a, 4b...4g) gebildet, die parallel zueinander ausgerichtet sind und einander berühren. Die Schlauchsegmente 4 gehen jeweils vom Versorgungsschlauch 2 aus und sind an diesen über die Verbindungen 8 (8a, 8b...8g) so angeschlossen, dass sie mit Druck beaufschlagt werden können. Sie erstrecken sich vom Versorgungsschlauch 2 ringförmig zum Versorgungsschlauch 2 zurück, sind aber an ihrem jeweils anderen Ende nicht an den Versorgungsschlauch 2 angeschlossen, sondern tot gelegt. Gleichwohl sind diese Enden 9 (9a, 9b...9g) mit dem Versorgungsschlauch 2 über Verklebungen oder Verschweißungen fest verbunden. Alternativ können auch die Enden 9 an den Versorgungsschlauch 2 angeschlossen sein.

Die einzelnen Schlauchsegmente 4 sind an ihren Kontaktlinien ebenfalls miteinander verbunden, d. h. verklebt oder verschweißt. Dies bedeutet, dass das Schlauchsegment 4a an seiner Kontaktlinie mit Schlauchsegment 4b verschweißt ist, Schlauchsegment 4b an seiner Kontaktlinie mit Schlauchsegment 4c, usw. Die Schlauchsegmente bilden eine geschlossene Wandung aus und werden zentral über den Versorgungsschlauch jeweils über die Anschlussstellen 8a, 8b,...8g mit Druck beaufschlagt.

Die Herstellung des in Figur 1 gezeigten Schlauchballons kann beispielsweise dadurch erfolgen, dass man entsprechende Schlauchsegmente 4 an den Versorgungsschlauch 2 parallel zueinander anschweißt bzw. anschließt, nachdem der Versorgungsschlauch 2 an den entsprechenden Stellen mit Durchtritten versehen wurde. Der Versorgungsschlauch 2 wird am distalen Ende verschlossen. Die toten Enden 9 der Schlauchsegmente 4 werden dann ebenfalls mit dem Versorgungsschlauch 2 verklebt oder verschweißt.

Vorteilhaft kann die Herstellung dadurch erfolgen, dass zwei Ballonschläuche doppelt gelegt werden und am proximalen wie distalen Ende umlaufend verschweißt werden. Am proximalen Ende wird die Zuführung für die Druckbeaufschlagung, der Versorgungsschlauch 2, eingeschweißt. Über die Länge des Ballons wird in definierten Abständen radial geschweißt, so dass einzelne Schlauchsegmente entstehen. In Fortsetzung des angeschweißten Versorgungsschlauchs 2 bleibt ein "Flutkanal" (Inflationslumen) offen, d. h. in diesem Bereich wird nicht verschweißt.

Dadurch, dass der Ausgangsschlauch für die Herstellung auf sich selbst umgestülpt bzw. umgeschlagen wird, kann eine Schweißnaht, vorzugsweise die distale Schweißnaht, eingespart werden.

Die Wanddicke des Schlauchballons ergibt sich aus den Abständen der radialen Schweißungen. Hierdurch wird der Durchmesser der einzelnen, radial verlaufenden Schläuche bestimmt, der im allgemeinen, je nach Einsatzzweck, 0,5 bis 5 mm betragen kann.

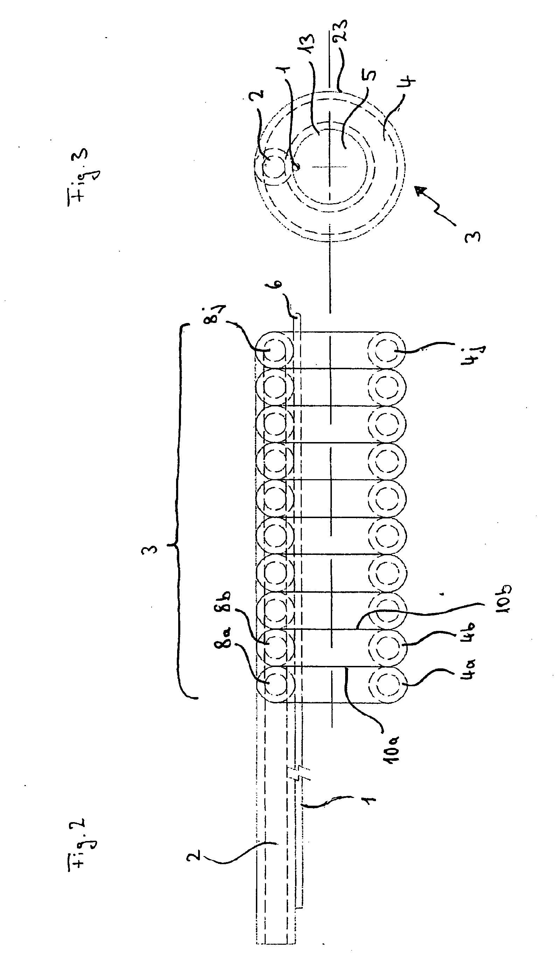

Figur 2 zeigt eine schematische Schnittzeichnung durch einen Schlauchballon gemäß Figur 1 in Längsrichtung. Der Versorgungsschlauch 2, bei dem mit durchbrochenen Linien die Wandstärke eingezeichnet ist, verläuft parallel zum Führungsdraht 1.

Vom Versorgungsschlauch 2 zweigen in dessen distalem Bereich, der der Länge des Schlauchballons 3 entspricht, in den Positionen acht Schlauchsegmente 4 ab, die über den Versorgungsschlauch 2 jeweils mit Druck beaufschlagt werden können. Die Schlauchsegmente 4 sind an ihren Kontaktlinien 10 miteinander verschweißt. Dies bedeutet, dass die Schlauchsegmente 4a und 4b an der Kontaktlinie 10a verschweißt sind, die Schlauchsegmente 4b und 4c an der Kontaktlinie 10b.

Der Führungsdraht 1 verläuft durch den Innenraum des Schlauchballons 3 und ragt mit seinem distalen Ende 6 über den Schlauchballon 3 hinaus. Zweckmäßigerweise ist er an der Innenseite des Schlauchballons 3 festgelegt. Der Führungsdraht kann im Bereich seines distalen Endes 6 und unmittelbar proximal zum Ballon 3 röntgendichte Marker aufweisen. Alternativ oder zusätzlich können auf dem Schlauchballon am proximalen und distalen Ende Marker vorgesehen sein.

Figur 3 zeigt eine weitere Schnittzeichnung senkrecht zum Verlauf des Versorgungsschlauchs der Ausführungsform von Figur 1/2. Die Schnittzeichnung zeigt den Schlauchballon 3 mit seiner inneren Wandung 13 und seiner äußeren Wandung 23 sowie, durch die gestrichelte Linie abgetrennt, den mit Druckmedium zu beaufschlagenden inneren Freiraum. Das Druckmedium wird durch den Versorgungsschlauch 2, der zur Pumpe führt, bereitgestellt. Entlang der Innenwandung 13, unmittelbar angrenzend und daran festgelegt, verläuft der Führungsdraht 1.

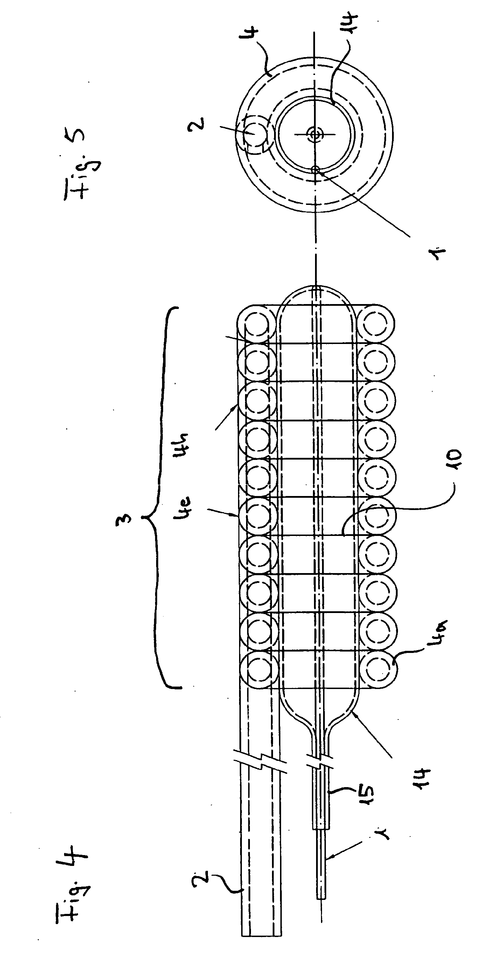

Figur 4 zeigt eine zweite Variante eines erfindungsgemäß zum Einsatz kommenden Schlauchballons 3, der in seinem Inneren (Hohlraum) einen inneren Ballon 14 aufweist. Wie zuvor besteht der Schlauchballon 3 aus einzelnen Schlauchsegmenten 4a, ... 4e, ...4h, die an ihren Kontaktlinien 10 miteinander verschweißt sind. Der Schlauchballon 3 steht in Verbindung mit einem Versorgungsschlauch 2, der ausgeführt, wie bereits zuvor beschrieben.

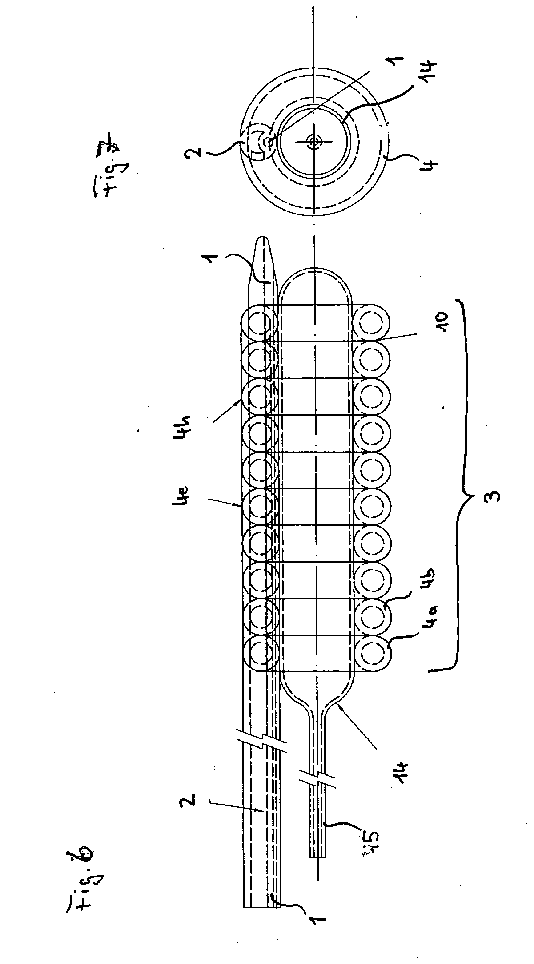

Der Innenballon 14 weist einen separaten Versorgungsschlauch 15, über den die Druckbeaufschlagung erfolgt. Der Führungsdraht 1 verläuft entlang der Innenwandung des Schlauchballons 3 an der Außenwandung des Innenballons 14, siehe Fig. 6.

Figur 5 ist eine Darstellung der Ausführungsform von Fig. 4 im Querschnitt. Das Schlauchsegment 4 umgibt konzentrisch den Innenballon 14 und steht in direktem Kontakt mit dem Führungsdraht 1, der entlang der Innenwandung der Schlauchsegmente 4 verläuft. Die Schlauchsegmente 4 stehen in Verbindung mit dem Versorgungsschlauch 2.

Figur 6 zeigt eine dritte Ausführungsform der Erfindung mit dem Schlauchballon 3, der über den Versorgungsschlauch 2 expandiert wird in dessen Innerem der Führungsdraht 1 verläuft. Die einzelnen Schlauchsegmente 4a, 4b, ...4e, ...4h sind an den Kontaktstellen 10 miteinander verschweißt. Im Inneren der Schlauchsegmente 4 verläuft ein Innenballon 14, der über einen eigenen Versorgungsschlauch 15 verfügt. Der Innenballon 14 weitet bei seiner Expansion den Schlauchballon 3 bzw. die Segmente 4 des Schlauchballons 3 auf und unterstützt somit deren Expansion.

Figur 7 ist eine Querschnittsdarstellung der Ausführungsform von Fig. 6 mit dem Schlauchsegment 4, dem Innenballon 14 und dem Versorgungsschlauch 2. Der Führungsdraht 1 verläuft in einer separaten Kammer des Versorgungsschlauches 2 unmittelbar angrenzend an den vom Innenballon 14 ausgefüllten Innenraum der Schlauchsegmente 4.

Die Varianten gemäß Fig. 4 bis 7 zeigen den erfindungsgemäßen Dilatationskatheter mit eingesetztem Innenballon 14. Es versteht sich, dass dieser Innenballon 14 nur ein Hilfsmittel ist, um den eigentlichen Schlauchballon 13 zur vollen Expansion zu bringen. Nachdem der Schlauchballon 3 voll expandiert ist, wird der Innenballon druckentlastet und kann aus dem Innenraum des Schlauchballons 3 herausgezogen werden. Damit wird dieser Innenraum für den Blutdurchgang frei gegeben.

## Patentansprüche

1. Ballondilatationskatheter, insbesondere für die perkutane transluminale Coronar-Angioplastie (PTCA), mit einem Führungsdraht (1), einem parallel zum Führungsdraht (1) verlaufenden Versorgungsschlauch (2) zum Aufpumpen eines Dilatationsballons (3) sowie dem in distalen Bereich des Versorgungsschlauches angeordneten Dilatationsballon (3), wobei der Dilatationsballon (3) schlauchförmig ausgebildet ist und einer Wandung aus einer Mehrzahl von aneinandergrenzenden Schlauchsegmenten (4),
**dadurch gekennzeichnet, dass** die Schlauchsegmente (4) vom Versorgungsschlauch (2) ausgehen und ringförmig zum Versorgungsschlauch (2) zurückführen.

2. Katheter nach Anspruch 1, **dadurch gekennzeichnet, dass** die Schlauchsegmente (4) an den Kontaktlinien (10) miteinander verbunden sind.

3. Katheter nach Anspruch 2, **dadurch gekennzeichnet, dass** die Schlauchsegmente (4) an den Kontaktlinien (10) miteinander verschweißt sind.

4. Katheter nach Anspruch 1, 2 oder 3 **dadurch gekennzeichnet, dass** jedes Schlauchsegment (4) über nur eine Verbindung (8) an den Versorgungsschlauch (2) angeschlossen ist.

5. Katheter nach einem der vorstehenden Ansprüche, **dadurch gekennzeichnet, dass** der schlauchförmig ausgebildete Dilatationsballon (3) um einen inneren Ballon (14) herumgeführt ist, der an einem separaten Versorgungsschlauch (15) angeschlossen ist und separat geführt werden kann.

6. Katheter nach Anspruch 5, **dadurch gekennzeichnet, dass** der innere Ballon (14) über einen separaten Führungsdraht verfügt.

7. Katheter nach einem der vorstehenden Ansprüche, **dadurch gekennzeichnet, dass** der Führungsdraht (1) innerhalb des schlauchförmigen Dilatationsballons (3) verläuft.

8. Katheter nach Anspruch 11, **dadurch gekennzeichnet, dass** der Führungsdraht (1) an der Innenwandung (13) des Ballons (3) festgelegt ist.

9. Katheter nach Anspruch 7, **dadurch gekennzeichnet, dass** der Führungsdraht (1) an der dem Versorgungsschlauch (2) gegenüberliegenden Seite der innenwandung (13) des Ballons (3) festgelegt ist

10. Katheter nach einem der vorstehenden Ansprüche, **dadurch gekennzeichnet, dass** Führungsdraht (1) und/oder Ballon (3) an ihrem proximalen und/oder distalen Ende Marker aufweisen.

11. Katheter nach einem der vorstehenden Ansprüche, **dadurch gekennzeichnet, dass** der Ballon (3) aus Polyethylenterephthalat, Polyamid oder Polyolefin besteht.

12. Katheter nach einem der vorstehenden Ansprüche, **dadurch gekennzeichnet, dass** Ballon (3) und Versorgungsschlauch aus unterschiedlich dehnbaren Materialien bestehen.

13. Katheter nach einem der vorstehenden Ansprüche, **dadurch gekennzeichnet, dass** der Katheter selbst aus einem im Wesentlichen druckfesten Material besteht.

14. Katheter nach einem der vorstehenden Ansprüche, **dadurch gekennzeichnet, dass** der Versorgungsschlauch (2) unmittelbar proximal zum Schlauchballon (3) mit einem Einwegventil ausgestattet ist, dass in Richtung auf den Schlauchballon (3) öffnet.

## Claims

1. A balloon dilation catheter, particularly for percutaneous transluminal coronary angioplasty (PTCA), with a guide wire (1), a supply tube (2), which extends parallel to the guide wire (1) for inflating a dilation balloon (3) and the dilation balloon (3) disposed in the distal region of the supply tube, wherein the dilation balloon (3) is of tubular shape, and a wall composed of a plurality of adjoining tube segments (4), **characterised in that** the tubes segments (4) start from the supply tube (2) and lead back in annular manner to the supply tube (2).

2. A catheter as claimed in Claim 1, **characterised in that** the tube segments (4) are connected together at the contact lines (10).

3. A catheter as claimed in Claim 2, **characterised in that** the tube segments (4) are welded together at the contact lines (10).

4. A catheter as claimed in Claims 1, 2 or 3, **characterised in that** each tube segment (4) is connected by means of only one connection (8) to the supply tube (2).

5. A catheter as claimed in one the preceding Claims, **characterised in that** the tube-shaped dilation balloon (3) extends around an inner balloon (14), which is connected to a separate supply tube (15) and can be guided separately.

6. A catheter as claimed in Claim 5, **characterised in that** the inner balloon (14) has a separate guide wire.

7. A catheter as claimed in one of the preceding Claims, **characterised in that** the guide wire (1) extends within the tubular dilation balloon (3).

8. A catheter as claimed in Claim 7, **characterised in that** the guide wire (1) is fastened to the inner wall (13) of the balloon (3).

9. A catheter as claimed in Claim 7, **characterised in that** the guide wire (1) is fastened to the side of the inner wall (13) of the balloon (3) opposite to the supply tube (2).

10. A catheter as claimed in one of the preceding Claims, **characterised in that** the guide wire (1) and/or balloon (3) have markers at their proximal and/or distal ends.

11. A catheter as claimed in one the preceding Claims, **characterised in that** the balloon (3) consists of polyethyrlene terephthalate polyamide or polyolefine.

12. A catheter as claimed in one of the preceding Claims, **characterised in that** the balloon (3) and supply tube consist of materials of different expansibility.

13. A catheter as claimed in one the preceding Claims, **characterised in that** the catheter itself consists of a substantially pressure-resistant material.

14. A catheter as claimed in one the preceding Claims, **characterised in that** the supply tube (2) is provided directly proximally of the tubular balloon (3) with a one-way valve, which opens in the direction of the tubular balloon (3).

## Revendications

1. Cathéter de dilatation à ballonnet, en particulier pour l'angioplastie coronarienne percutanée transluminale **(PTCA),** avec un fil de guidage **(1) ;** un tuyau d'alimentation **(2)** s'étendant parallèlement au fil de guidage **(1)** pour gonfler un ballonnet de dilatation **(3)** ainsi que le ballonnet de dilatation **(3)** placé dans la zone distale du tuyau d'alimentation, le ballonnet de dilatation **(3)** étant réalisé en forme de tuyau et présentant une paroi constituée d'une pluralité de segments de tuyau **(4)** contigus, **caractérisé en ce que** les segments de tuyau **(4)** partent du tuyau d'alimentation **(2)** et reviennent de manière annulaire jusqu'au tuyau d'alimentation **(2).**

2. Cathéter selon la revendication 1, **caractérisé en ce que** les segments de tuyau **(4)** sont reliés entre eux au niveau des lignes de contact **(10).**

3. Cathéter selon la revendication 2, **caractérisé en ce que** les segments de tuyau **(4)** sont soudés entre eux au niveau des lignes de contact **(10).**

4. Cathéter selon la revendication 1, 2 ou 3, **caractérisé en ce que** chaque segment de tuyau **(4)** n'est raccordé au tuyau d'alimentation **(2)** que par une seule jonction **(8).**

5. Cathéter selon l'une des revendications précédentes, **caractérisé en ce que** le ballonnet de dilatation **(3)** en forme de tuyau passe autour d'un ballonnet intérieur **(14)** qui est raccordé à un tuyau d'alimentation séparé **(15)** et peut être passé séparément.

6. Cathéter selon la revendication 5, **caractérisé en ce que** le ballonnet intérieur **(14)** dispose d'un fil de guidage séparé.

7. Cathéter selon l'une des revendications précédentes, **caractérisé en ce que** le fil de guidage **(1)** s'étend à l'intérieur du ballonnet de dilatation **(3)** en forme de tuyau.

8. Cathéter selon la revendication 11, **caractérisé en ce que** le fil de guidage **(1)** est fixé sur la paroi intérieure **(13)** du ballonnet **(3).**

9. Cathéter selon la revendication 7, **caractérisé en ce que** le fil de guidage **(1)** est fixé au côté de la paroi intérieure **(13)** du ballonnet **(3)** opposé au tuyau d'alimentation **(2).**

10. Cathéter selon l'une des revendications précédentes, **caractérisé en ce que** le fil de guidage **(1)** et/ou le ballonnet **(3)** présentent des marqueurs à leur extrémité proximale et/ou distale.

11. Cathéter selon l'une des revendications précédentes, **caractérisé en ce que** le ballonnet **(3)** est constitué de polyéthylène-téréphthalate, de polyamide ou de polyoléfine.

12. Cathéter selon l'une des revendications précédentes, **caractérisé en ce que** le ballonnet **(3)** et le tuyau d'alimentation sont constitués de matériaux extensibles différemment.

13. Cathéter selon l'une des revendications précédentes, **caractérisé en ce que** le cathéter lui-même est constitué d'un matériau pour l'essentiel à l'épreuve de la pression.

14. Cathéter selon l'une des revendications précédentes, **caractérisé en ce que** le tuyau d'alimentation **(2)** est muni, de manière directement proximale au ballonnet tubulaire **(3),** d'une vanne à une voie qui s'ouvre en direction du ballonnet tubulaire **(3).**
